# EUROPÄISCHE PATENTANMELDUNG

(11) **EP 0 816 333 A1**
(43) Veröffentlichungstag der Anmeldung: **07.01.1998**
(21) Anmeldenummer: 97110398.1
(22) Anmeldetag: 25.06.1997
(51) Int. Cl.: C07C 263/10, C07C 263/20

(54) **Verfahren zur Herstellung von Lösungen eines oder mehrerer Isocynate, die keine nennenswerten Mengen an farbgebenden Komponenten aufweisen, sowie die Verwendung der so erhaltenen Isocyanate**

(30) Priorität: 26.06.1996 DE 19625644
(71) Anmelder: BASF AKTIENGESELLSCHAFT, 67056 Ludwigshafen (DE)
(72) Erfinder: Kraus, Rupert, Dr., 67065 Ludwigshafen (DE); Tesch, Helmut, Dr., 67127 Rödersheim-Gronau (DE); Bruchmann, Bernd, Dr., 67069 Ludwigshafen (DE); Seyfert, Wilfried, Dr., 67273 Weisenheim (DE); Nevejans, Filip, 9170 St. Pauwels (BE); Van den Abeel, Peter, 2950 Kapellen (BE)
(74) Vertreter: Isenbruck, Günter, Dr.

(57) **Zusammenfassung**

Verfahren zur Herstellung von Lösungen, enthaltend mindestens ein Isocyanat, wobei die oben definierte Lösung nach der Phosgenierung und vor der vollständigen Abtrennung des Lösungsmittels und von Restphosgen einer Behandlung mit Wasserstoff unterzogen wird, ein Verfahren zur Herstellung von Isocyanaten oder Isocyanat-Gemischen, sowie die Verwendung der so hergestellten Isocyanate zur Herstellung von Polyurethanen, insbesondere von Polyurethan-Schaumstoffen.

## Beschreibung

Die Erfindung betrifft ein Verfahren zur Herstellung von Lösungen, enthaltend mindestens ein Isocyanat, wobei die oben definierte Lösung nach der Phosgenierung und vor der vollständigen Abtrennung des Lösungsmittels und von Restphosgen einer Behandlung mit Wasserstoff hydrierende Behandlung unterzogen wird, ein Verfahren zur Herstellung von Isocyanaten oder Isocyanat-Gemischen, sowie die Verwendung der so hergestellten Isocyanate zur Herstellung von Polyurethanen, insbesondere von Polyurethan-Schaumstoffen. Dabei ist zu beachten, daß die erhaltenen Isocyanate keine nennenswerten Mengen an farbgebenden Komponenten enthalten, einen niedrigen Gehalt an hydrolisierbarem Chlor aufweisen und verglichen mit den Isocyanten gemäß des Standes der Technik eine hellere Farbe besitzen.

Isocyanate und Isocyanatgemische werden nach bekannten Verfahren durch Phosgenierung der entsprechenden Amine hergestellt. Für Polyurethanschäume finden beispielsweise di- oder polyfunktionelle aromatische Isocyanate der Diphenylmethandiisocyanat-Reihe (MDI) Anwendung. Bedingt durch den Herstellungsprozeß werden nach der Phosgenierung und der anschließenden Aufarbeitung (Abtrennung des Lösungsmittels; Abdestillieren von monomerem MDI) oft verhältnismäßig dunkel gefärbte Produkte erhalten, die wiederum gelblich verfärbte Polyurethan(PUR)-Schäume oder andere PUR-Materialien ergeben. Dies ist unerwünscht, da die Färbung geringfügige Inhomogenitäten hervortreten läßt, z.B. als Schlieren in den erhaltenen Schäumen. Helle Isocyanate, bzw. Isocyanate, die eine reduzierte Menge an farbgebenden Komponenten enthalten, werden deshalb als Rohstoffe bevorzugt. Es hat daher nie an Versuchen gefehlt, Polyisocyanate, insbesondere solche der Diphenylmethandiisocyanat-Reihe, mit möglichst heller Farbe zu erhalten. Zur empirischen Farbaufhellung von MDI sind zahlreiche Methoden bekannt. Die Natur der störenden Farbkörper ist bisher nur unzureichend geklärt.

Die bislang bekannten Verfahren lassen sich grob in vier Gruppen unterteilen:

### 1. Verfahren, bei denen des Ausgangsmaterial Diaminodiphenylmethan (MDA) einer Behandlung und/oder Reinigung unterzogen wurde

Die EP-A-0 546 398 beschreibt ein Verfahren zur Herstellung von Polymer-MDI, in dem das als Edukt verwendete Polymethylenpolyphenylpolyamin vor der Phosgenierung angesäuert wird.

Die EP-A-0 446 781 betrifft ein Verfahren zur Herstellung von Diaminodiphenylmethanen, die zuerst mit Wasserstoff behandelt und anschließend einer Phosgenierung unterzogen werden, wobei ein helleres MDI erhalten wird.

Die oben genannten Verfahren ergeben nur eine geringfügige Farbverbesserung, da die Farbkörper im MDI erfahrungsgemäß nicht nur aus bestimmten MDA-Nebenkomponenten entstehen, sondern auch aus Farbvorläufern resultieren, die durch Nebenreaktionen während der Phosgenierung gebildet werden.

### 2. Verfahrenstechnische Lösungen im Phosgenierungsprozeß

Die US-A 5 364 958 betrifft ein Verfahren zur Herstellung von Polyisocyanaten, demgemäß nach der Phosgenierung das Phosgen bei niedriger Temperatur vollständig entfernt wird und anschließend das Isocyanat in der Wärme mit HCl-Gas behandelt wird.

Die EP-A-0 581 100 betrifft ein Verfahren zur Herstellung von Polyisocyanaten, in dem nach der Phosgenierung ein chemisches Reduktionsmittel vor dem Abtrennen von Lösungsmittel zugegeben wird, wobei gemäß dieser Patentschrift ebenfalls hellere Produkte erhalten werden.

Diese Verfahren versuchen zwar an der richtigen Stelle die Verfärbungen verursachenden Komponenten abzutrennen, sie sind jedoch aufgrund ihres hohen technischen Aufwands bzw. der hohen Kosten in ihrem Farbaufhellungseffekt zu wenig effizient, da der Abbau von Farbvorläufern durch unvollständig ablaufende chemische Reaktionen nur in geringem Umfang erfolgt.

### 3. Zusatz von Farbaufhellungs-Additiven zum nach der Phosgenierung und vor der Aufarbeitung erhaltenen Isocyanat-Rohprodukt

Gemäß der US-A 4 465 639 wird dem nach der Phosgenierung erhaltenen Rohprodukt zur Farbaufhellung Wasser zugesetzt. Zum gleichen Zweck beschreiben die EP-A-0 538 500, EP-A-0 445 602 und EP-A-0 467 125 den Zusatz von Carbonsäuren, Alkanolen bzw. Polyetherolen nach der Phosgenierung.

Die oben beschriebenen Verfahren zur Farbaufhellung sind zwar effizient, sie weisen jedoch Nachteile dahingehend auf, daß neben der Farbaufhellung die zugegebenen Additive allesamt Reaktionen mit den als Produkt anfallenden Isocyanaten eingehen.

### 4. Nachbehandlung des Endprodukts

Die EP-A-0 133 538 beschreibt die Reinigung von Isocyanaten durch Extraktion, wodurch Fraktionen eines hellen MDI erhalten werden.

Die EP-A-0 561 225 betrifft ein Verfahren zur Herstellung von Isocyanaten oder Isocyanatgemischen, die laut dieser Schrift keine farbgebenden Komponenten aufweisen, wobei die Isocyanate nach der Phosgenierung der entsprechenden Amine einer Wasserstoffbehandlung bei einem Druck von 3 bis 150 bar und einer Temperatur von 100 bis 180°C unterworfen werden. Dabei werden gemäß den dort beschriebenen Beispielen Isocyanat-Endprodukte als solche oder in Form ihrer Lösungen in geeigneten Lösungsmitteln hydriert.

Diese farbverbessernden Nachbehandlungen der Isocyanat-Endprodukte nach der vollständigen Abtrennung des Lösungsmittels bei erhöhter Temperatur sind ebenfalls wenig effizient, da durch die hohen Temperaturen, die bei der Aufarbeitung, insbesondere dem Abdestillieren des Lösungsmittels und (bei der Herstellung von Polymer-MDI) dem Abtrennen von monomeren MDI auftreten, bereits stabile Farbkörper entstanden sind, die chemisch kaum noch angreifbar sind.

Der vorliegenden Erfindung liegt demgemäß die Aufgabe zugrunde, ein neues Verfahren zur Herstellung von Lösungen, die Isocyanate enthalten, die keine nennenswerten Mengen an farbgebenden Komponenten aufweisen, verglichen mit den aus dem Stand der Technik bekannten Isocyanaten eine hellere Farbe besitzen und einen relativ niedrigen Gehalt an hydrolysierbarem Chlor aufweisen, bereitzustellen, wobei dieses Verfahren auch als Teil des Gesamtverfahrens zur Herstellung von Isocyanaten ausgehend von den entsprechenden Aminen einzubeziehen sein sollte.

Diese Aufgabe konnte überraschenderweise durch das Verfahren zur Herstellung einer Lösung, enthaltend mindestens ein Isocyanat, gemäß der vorliegenden Erfindung gelöst werden, das dadurch gekennzeichnet ist, daß eine Lösung, enthaltend mindestens ein Isocyanat, nach der Phosgenierung und vor der vollständigen Abtrennung des Lösungsmittels einer Behandlung mit Wasserstoff unterzogen wird. Erfindungsgemäß wird so das Isocyanat nach der Phosgenierung des entsprechenden Amins nach der weitgehenden Abtrennung von Restphosgen aus der Lösung ohne weitere Aufarbeitung, d.h. ohne zusätzliche Temperaturbelastung, direkt bei verhältnismäßig niedrigen Temperaturen und Drücken einer Behandlung mit Wasserstoff unterzogen.

Ferner betrifft die vorliegende Erfindung ein Verfahren zur Herstellung eines Isocyanats oder eines Gemischs aus zwei oder mehr Isocyanaten, das dadurch gekennzeichnet ist, daß nach der Phosgenierung des entsprechenden Amins und vor der prozeßüblichen Abtrennung des Lösungsmittels und der thermischen Nachbehandlung eine Behandlung mit Wasserstoff, wie im Rahmen der vorliegenden Anmeldung definiert, durchgeführt wird.

Sofern MDI verwendet wird, kann gegebenenfalls monomeres MDI abdestilliert werden, wobei Polymer-MDI, das keine nennenswerten farbgebenden Komponenten aufweist, zurückbleibt.

Der im Rahmen der vorliegenden Erfindung verwendete Begriff "Isocyanat, das keine nennenswerten Mengen an farbgebenden Komponenten aufweist", bedeutet, daß erfindungsgemäß Lösungen von Isocyanaten bzw. Isocyanate erhalten werden, deren Anteil an farbgebenden Komponenten im allgemeinen so gering ist, daß die Iod-Farbzahl (IFZ), gemessen nach DIN 6162, bei höchstens ungefähr 70, vorzugsweise bei höchstens ungefähr 40, und insbesondere bei ungefähr 20 bis ungefähr 30 liegt.

Ferner weisen die erfindungsgemäß hergestellten Isocyanate einen Gehalt an hydrolysierbarem Chlor von weniger als 1500 ppm, vorzugsweise weniger als 1000 ppm und insbesondere weniger als 800 ppm, gemessen nach ASTM D 4663-87, auf. Der obige Begriff "*hydrolysierbares Chlor*" steht für den Chlorgehalt der Isocyanate, der aus Verbindungen stammt, die bei der Hydrolyse Chloridionen freisetzen, wie z.B. Phosgen, HCl oder primäre und sekundäre Carbamoylchloride.

Im Rahmen des erfindungsgemäßen Verfahrens können prinzipiell Lösungen aller Isocyanate, die durch Phosgenierung der entsprechenden Amine hergestellt werden, eingesetzt werden. Beispielhaft zu nennen sind: 1,6-Hexamethylendiisocyanat, Isophorondiisocyanat, Cyclohexylisocyanat, Phenylisocyanat, 4-Toluylisocyanat, 1,5-Naphtylendiisocyanat, 2,4- oder 2,6-Toluylendiisocyanat oder deren Gemische, 4,4'-, 2,4'- oder 2,2'-Diphenylmethandiisocyanat oder deren Gemische, sowie mehrkernige oder höhermolekulare (Polymer-)Derivate der obigen Diisocyanate.

Unter diesen sind Diphenylmethandiisocyanate (MDI) und insbesondere Polymer-MDI bevorzugt.

Als Lösungsmittel können alle für den Phosgenierungsprozeß bekannten, inerten aromatischen, aliphatischen oder alicyclischen Kohlenwasserstoffe bzw. Halogenkohlenwasserstoffe eingesetzt werden, in denen das jeweilige Isocyanat löslich ist, und die unter den Reaktionsbedingungen der Phosgenierung und der Behandlung mit Wasserstoff nicht angegriffen werden. Beispiele solcher Lösungsmittel sind aromatische Verbindungen, wie z.B. Mono- oder Dichlorbenzol, Toluol, Xylole und Naphthalinderivate, Alkane mit 5 bis 12 Kohlenstoffatomen, wie z.B. Hexan, Heptan, Octan, Nonan, Decan, Cycloalkane, wie z.B. Cyclohexan, inerte Ester und inerte Ether, wie z.B. Ethyl- oder Butylacetat, Tetrahydrofuran, Dioxan oder Diphenylether.

Die der hydrierenden Behandlung unterzogene Lösung mindestens eines Isocyanats enthält nach der Phosgenierung und der weitgehenden Abtrennung von Phosgen und HCl, die im allgemeinen dem erfindungsgemäßen Verfahren vorausgehen, noch Spuren von Phosgen und HCl, die jedoch für den Prozeß unkritisch sind, wobei HCl-Spuren in der Lage sein können, die Aktivität des für die Behandlung mit Wasserstoff eingesetzten Katalysators zu erhöhen. Vorzugsweise wird die Behandlung mit Wasserstoff direkt im während der Phosgenierung eingesetzten Lösungsmittel durchgeführt.

Die Reaktionsbedingungen der Behandlung mit Wasserstoff sind im allgemeinen wie folgt:

Die Konzentration des Isocyanats im Lösungsmittel beträgt im allgemeinen ungefähr 1 bis ungefähr 90 Gew.-%, vorzugsweise ungefähr 5 bis ungefähr 50 Gew.-%.

Die Temperatur während der Hydrierung liegt im allgemeinen zwischen ungefähr 25 und ungefähr 200° C und vorzugsweise zwischen ungefähr 70 und 150° C.

Der während der Hydrierung verwendete Druck liegt im allgemeinen zwischen ungefähr 2 x 10⁴ und ungefähr 3 x 10⁷ Pa, vorzugsweise zwischen ungefähr 5 x 10⁴ und ungefähr 2,8 x 10⁵ Pa, und insbesondere zwischen ungefähr 1 x 10⁵ und ungefähr 2,5 x 10⁵ Pa.

Die Hydrierung wird im allgemeinen über einen Zeitraum von einer Minute bis zu drei Stunden und vorzugsweise von fünf Minuten bis sechzig Minuten durchgeführt.

Als Katalysatoren kommen alle bekannten heterogenen Hydrierungskatalysatoren in Frage, die unter stark sauren, wasserfreien Bedingungen (HCl) stabil sind und keine Reaktionen mit Isocyanaten eingehen. Dabei kommen insbesondere die zur Hydrierung bekannten Übergangsmetalle und Übergangsmetallverbindungen der I., VII. und VIII. Nebengruppe des Periodensystems in Frage, wobei vorzugsweise die Edelmetalle, wie z.B. Pd, Pt, Rh, Ru usw. verwendet werden. Diese werden nach bekannten Verfahren aktiviert und können auch als geträgerte Katalysatoren vorliegen, wobei als Trägermaterialien Aktivkohlen, Aluminiumoxide und Silicate bevorzugt sind. Dabei liegt der Gehalt der Aktivkomponente im allgemeinen zwischen ungefähr 0,5 bis ungefähr 30 Gew.-%, vorzugsweise zwischen ungefähr 2 bis ungefähr 10 Gew.-%, jeweils bezogen auf das Gesamtgewicht des Katalysators. Bezogen auf das eingesetzte Isocyanat beträgt die Menge des Katalysators im allgemeine ungefähr 0,01 bis ungefähr 20 Gew.-%, vorzugsweise ungefähr 0,5 bis ungefähr 10 Gew.-%.

Die hydrierende Behandlung kann praktisch drucklos oder unter Überdruck nach den bekannten Verfahren der kontinuierlichen und der diskontinuierlichen Hydrierung durchgeführt werden. So kann die Isocyanatlösung vor dem Kontakt mit dem Katalysator mit Wasserstoff gesättigt werden, oder es kann Wasserstoff nach der Katalysatorzugabe der Lösung des mindestens einen Isocyanats aufgepreßt werden. Die Hydrierung kann kontinuierlich oder diskontinuierlich in Suspension des Katalysators oder auch kontinuierlich in Rieselphase über ein Katalysatorbett erfolgen. Die Durchmischung bei der Suspensionsfahrweise kann z.B. mittels üblicher Rührer, Begasungsrührer oder auch durch statische Mischer erfolgen. Kontinuierlich kann die Hydrierung in Suspensionsfahrweise in einem Kreislaufsystem über Düseneinspeisung mit Ausschleusung des katalysatorfreien Produkts nach bekannten Verfahren erfolgen. Bei der kontinuierlichen Fahrweise über ein Katalysatorbett kann z.B. im H₂-Gleich- oder -Gegenstrom gearbeitet werden.

Die Abtrennung und Rückführung des suspendierten Katalysators erfolgt gemäß den im Stand der Technik bekannten Verfahren zur Abtrennung feinverteilter Feststoffe aus Lösungen.

Die erhaltene Lösung des mindestens einen Isocyanats im inerten Lösungsmittel kann anschließend in der prozeßüblichen Weise durch sukzessives Abdestillieren des Lösungsmittels über mehrere Kolonnen bei abnehmendem Druck aufgearbeitet werden. Zuletzt läßt sich durch kurzzeitiges Erhitzen auf hohe Temperaturen im Vakuum ein lösungsmittelfreies Roh-Isocyanat erhalten, welches einer anschließenden Destillation unterworfen werden kann, wobei beispielsweise monomeres MDI vom drei- und vierkernigen sowie höhermolekularem Polymer-MDI abgetrennt werden kann.

Somit betrifft die vorliegende Erfindung auch ein Verfahren zur Herstellung von Isocyanaten oder Gemischen aus zwei oder mehr Isocyanaten, in dem als eine Reaktionsstufe eine Behandlung mit Wasserstoff einer Lösung mindestens eines Isocyanats, wie im Rahmen der vorliegenden Anmeldung ausführlich beschrieben, durchgeführt wird.

Die nach dem erfindungsgemäßen Verfahren hergestellten Isocyanate oder Gemische aus zwei oder mehr Isocyanaten werden zur Herstellung von Polyurethanen mit vergleichsweise hellerer Farbe, vorzugsweise Polyurethan-Schaumstoffen verwendet.

Gegenüber den Verfahren des Standes der Technik zeigen die Verfahren gemäß der vorliegenden Erfindung unter anderem folgende Vorteile:

Durch die erfindungsgemäße Behandlung mit Wasserstoff auf der erwähnten Prozeßstufe werden Lösungen von Isocyanaten erhalten, die keine nennenswerten Mengen an farbgebenden Komponenten aufweisen, die dann zu deutlich helleren Isocyanaten führen.

Das erhaltene Produkt ist frei von Additiven.

Die erfindungsgemäße Behandlung mit Wasserstoff, die vorzugsweise direkt im bei der Phosgenierung verwendeten Lösungsmittel selbst durchgeführt wird, kann prinzipiell so in einem Verfahren zur Herstellung von Isocyanaten eingebaut werden, daß das gesamte Verfahren in kontinuierlicher Arbeitsweise durchgeführt werden kann.

Ferner ist das erfindungsgemäße Verfahren relativ energie- und ressourcensparend, da insgesamt weniger Temperaturänderungen erforderlich sind und unter verhältnismäßig niedrigen Drücken und bei mäßigen Temperaturen gearbeitet werden kann. Darüber hinaus entfällt eine nachträgliche Behandlung und Reinigung des als Endprodukt zu erhaltenden Isocyanats.

Die erhaltene Lösung mindestens eines Isocyanats, bzw. die erhaltenen Isocyanate weisen einen vergleichsweise niedrigen Gehalt an hydrolisierbaren Chlorverbindungen, wie oben definiert, die durch die Behandlung mit Wasserstoff in überwiegendem Maße ebenfalls zerstört werden, auf.

Das Verfahren besitzt eine hohe Effizienz, da Vorstufen von farbgebenden Substanzen abgebaut werden, und es demzufolge nicht erforderlich ist, thermodynamisch stabile Farbkörper an sich zu zerstören.

Das erfindungsgemäße Verfahren soll nunmehr anhand von einigen Beispielen näher erläutert werden.

### BEISPIELE

### Herstellung der MDI-Proben (Ansatz A)

150 g Diaminodiphenylmethan (MDA)-Rohbase, gelöst in 1,3 l Monochlorbenzol wurden in einem Rührreaktor mit einem Fassungsvermögen von 6 l mit 300 g Phosgen, gelöst in 1,3 l Monochlorbenzol, bei 50 bis 80°C drucklos umgesetzt. Die Temperatur wurde über 1 bis 2 h auf ungefähr 120°C erhöht, wobei die Umsetzung zum Isocyanat (ungefähr 180 g) erfolgte. Anschließend wurde unter schonenden Bedingungen (80°C, 0,2 x 10⁵ Pa) Restphosgen und 50 bis 95% des Monochlorbenzols abdestilliert. Das Reaktionsgemisch, das noch Spuren an Phosgen enthielt, wurde mit frischem Lösungsmittel (Monochlorbenzol oder ein anderes geeignetes Lösungsmittel) auf ungefähr 1,5 bis 2 l aufgefüllt, bis eine ca. 10%ige Lösung des Isocyanats im Lösungsmittel erhalten wurde. Dieses Gemisch wurde zur hydrierenden Behandlung eingesetzt.

### Hydrierende Behandlung der Diphenylmethandiisocyanat (MDI)-Proben

### Beispiel 1

Ein mit Monochlorbenzol auf 2 l aufgefüllter Ansatz A wurde in einem Druckreaktor mit einem Fassungsvermögen von 4 l, der mit einem Rührer versehen war, unter Zusatz von 10 g Pd/C (5 Gew.-% Palladium) 1 h lang bei 80° C unter einem H₂-Druck von 5 x 10⁵ Pa gerührt. Nach dem Entspannen wurde die Probe abgelassen und der Katalysator quantitativ abfiltriert. Das Lösungsmittel wurde am Rotationsverdampfer im Vakuum schonend abgezogen, und die Probe anschließend zweimal je 45 min lang bei 180° C und 0,01 x 10⁵ Pa am Rotationsverdampfer zum Entfernen von Lösungsmittelspuren nachbehandelt. Die Ergebnisse sind in Tab. I angeführt.

### Beispiel 2

Ein mit Toluol auf 2 l aufgefüllter Ansatz A mit einem Restgehalt an Monochlorbenzol von ca. 200 ml wurde in einem Druckreaktor mit einem Fassungsvermögen von 4 l, der mit einem Rührer versehen war, unter Zusatz von 10 g Pd/C (5 Gew.-% Palladium) 30 min lang bei 120° C und einem H₂-Druck von 2 x 10⁵ Pa behandelt. Nach dem Entspannen wurde die Probe abgelassen und der Katalysator quantitativ abfiltriert. Das Lösungsmittel wurde am Rotationsverdampfer im Vakuum schonend abgezogen, und die Probe anschließend einmal 45 min lang bei 180° C und 0,01 x 10⁵ Pa am Rotationsverdampfer nachbehandelt. Die Ergebnisse sind in Tab. I gezeigt.

### Beispiel 3

Ein mit Monochlorbenzol auf 2 l aufgefüllter Ansatz A wurde in einem Druckreaktor mit einem Fassungsvermögen von 4 l, der mit einem Rührer versehen war, unter Zusatz von 5 g Pd/C (5 Gew.-% Palladium) 1 h lang bei 80° C unter einem H₂-Druck von 2 x 10⁵ Pa behandelt. Nach dem Entspannen wurde die Probe abgelassen, und der Katalysator quantitativ abfiltriert. Das Lösungsmittel wurde am Rotationsverdampfer im Vakuum schonend abgezogen, und die Probe anschließend einmal 45 min lang bei 180° C und 0,01 x 10⁵ Pa am Rotationsverdampfer nachbehandelt. Die Ergebnisse sind in Tab. I gezeigt.

### Beispiel 4

Ein mit Monochlorbenzol auf 2 l aufgefüllter Ansatz A wurde in einem Druckreaktor mit einem Fassungsvermögen von 4 l, der mit einem Rührer versehen war, unter Zusatz von 10 g Pd/Al₂O₃ (5 Gew.-% Palladium) 30 min lang bei 120° C unter einem H₂-Druck von 2 x 10⁵ Pa behandelt. Nach dem Entspannen wurde die Probe abgelassen und der Katalysator quantitativ abfiltriert. Das Lösungsmittel wurde am Rotationsverdampfer im Vakuum schonend abgezogen und anschließend die Probe einmal 45 min lang bei 180° C und 0,01 x 10⁵ Pa am Rotationsverdampfer nachbehandelt. Die Ergebnisse sind in Tab. I gezeigt.

### Vergleichsbeispiel (VB) 1 (gemäß EP-A-0 561 225)

Ansatz A wurde im Vakuum vom Lösungsmittel (Monochlorbenzol) befreit und 45 min lang bei 180° C und 0,01 x 10⁵ Pa am Rotationsverdampfer behandelt. Es wurden ca. 175 bis 180 g Isocyanat-Produkt erhalten. Dieses wurde wiederum vollständig in Monochlorbenzol gelöst und in einem Druckreaktor mit einem Fassungsvermögen von 4 l, der mit einem Rührer versehen war, unter Zusatz von 10 g Pd/C (5 Gew.-% Palladium) 1 h lang bei 80° C unter einem H₂-Druck von 5 x 10⁵ Pa behandelt. Nach dem Entspannen wurde die Probe abgelassen und der Katalysator quantitativ abfiltriert. Das Lösungsmittel wurde im Rotationsverdampfer im Vakuum schonend abgezogen, und die Probe anschließend nochmals 45 min lang bei 180° C und 0,01 x 10⁵ Pa am Rotationsverdampfer rachbehandelt. Die Ergebnisse sind in Tab. I gezeigt.

### Vergleichsbeispiel 2 (ohne Wasserstoffbehandlung)

Ansatz A wurde im Vakuum vom Lösungsmittel (Monochlorbenzol) befreit und einmal 45 min lang bei 180° C und 0,01 x 10⁵ Pa am Rotationsverdampfer behandelt. Es wurden 175 g Isocyanat-Endprodukt erhalten. Die Ergebnisse sind in Tab. I gezeigt.

Es wurden die Kenndaten der gemäß den erfindungsgemäßen Beispielen 1 bis 4 und den Vergleichsbeispielen 1 und 2 erhaltenen Isocyanate bestimmt. Dabei wurde speziell die für MDI üblicherweise angegebene Jod-Farbzahl bestimmt. Hierzu wurden die Proben (verdünnt in Monochlorbenzol; Verhältnis Isocyanat : Monochlorbenzol 1:5) sowohl mit einem Komparatorgerät (Fa. Hellige) durch Vergleich mit Farbscheiben (entsprechend bestimmten Jod-Farbzahlen) untersucht, als auch Farbzahlen mit einem Photometer (Fa. Dr. Lange, Berlin) im IFZ-Programm-Modus bestimmt, wobei die erhaltenen Werte mit einem Faktor nach Eichung mit Jod-Standardlösung nach DIN 6162 korrigiert wurden.

**Tab. 1**

| **Kenndaten der Beispiele** | | | | | |
|---|---|---|---|---|---|
| **Bez.** | **NCO (%)** | **EHC** **(*)** **(ppm)** | **DHC****(**)** **(ppm)** | **IFZ 1:5 Neokomp.** | **IFZ 1:5 Photom.** |
| B1 | 31,2 | 89 | 525 | 25 | 14 |
| B2 | 30,5 | 72 | 471 | 18 | 8 |
| B3 | 31,2 | 93 | 497 | 20 | 8 |
| B4 | 31,3 | 148 | 577 | 30 | 21 |
| VB1 | 29,5 | 92 | 512 | 70 | 64 |
| VB2 | 31,9 | 413 | 998 | 70 | 29 |

| | | | | | |
|---|---|---|---|---|---|
| (*) EHC = Azidität ("easily hydrolysable chlorine"), gemessen entprechend ASTM D 4667-87 | | | | | |
| (**) DHC = Hydrolysierbares Chlor ("difficultly hydrolysable chlorine"), gemessen nach ASTM D 4663-87. NCO = Isocyanatgehalt, gemessen nach DIN 53185. | | | | | |

Darüber hinaus wurde eine Farbzahlbestimmung für unterschiedliche Farbtönungen durch Messung der Absorbtionswerte bei unterschiedlichen Wellenlängen durchgeführt (analog EP-A-0 676 391 A1). Hierzu wurden 2 g Produkt in 100 ml Monochlorbenzol gelöst und in 1 cm Rechteckküvetten aus Quarzglas vermessen.

**Tab. 2**

| **Messung der Absorbtionswerte** | | | |
|---|---|---|---|
| **Bez.** | **A[430nm]** | **A[520nm]** | **A[600nm]** |
| B1 | 0,046 | 0,008 | 0,002 |
| B2 | 0,045 | 0,007 | 0,002 |
| B3 | 0,159 | 0,014 | 0,003 |
| VB1 | 0,394 | 0,043 | 0,013 |
| VB2 | 0,163 | 0,031 | 0,011 |

Die Ergebnisse zeigen eine sehr gute Farbaufhellung von Roh-MDI durch hydrierende Behandlung bei gleichzeitig deutlicher Reduzierung der Werte für hydrolysierbares Chlor. Der NCO-Gehalt wird nur unwesentlich reduziert.

## Patentansprüche

1. Verfahren zur Herstellung einer Lösung, enthaltend mindestens ein Isocyanat, dadurch gekennzeichnet, daß eine Lösung enthaltend mindestens ein Isocyanat nach der Phosgenierung und vor der vollständigen Abtrennung des Lösungsmittels einer Behandlung mit Wasserstoff unterzogen wird.

2. Verfahren nach Anspruch 1, dadurch gekennzeichnet, daß die Behandlung mit Wasserstoff bei Drücken von 0,5 x 10⁵ bis 2,8 x 10⁵ Pa durchgeführt wird.

3. Verfahren nach Anspruch 1 oder 2, dadurch gekennzeichnet, daß die Behandlung mit Wasserstoff bei einer Temperatur von 70 bis 150° C durchgeführt wird.

4. Verfahren nach mindestens einem der vorstehenden Ansprüche, dadurch gekennzeichnet, daß die Behandlung mit Wasserstoff im Lösungsmittel der Phosgenierung durchgeführt wird.

5. Verfahren nach mindestens einem der vorstehenden Ansprüche, dadurch gekennzeichnet, daß das mindestens eine Isocyanat ein Isocyanat der Diphenylmethandiisocyanate (MDI)-Reihe ist.

6. Verfahren zur Herstellung eines Isocyanats oder eines Gemischs aus zwei oder mehr Isocyanaten, dadurch gekennzeichnet, daß eine Behandlung mit Wasserstoff, wie in mindestens einem der Ansprüche 1 bis 5 definiert, durchgeführt wird.

7. Verfahren nach Anspruch 6, dadurch gekennzeichnet, daß es kontinuierlich durchgeführt wird.

8. Verfahren nach Anspruch 6 oder 7, dadurch gekennzeichnet, daß als Produkt Polymer-MDI erhalten wird.

9. Verwendung eines Isocyanats oder eines Gemisches aus zwei oder mehr Isocyanaten, die mittels eines Verfahrens gemäß einem der Ansprüche 6 bis 8 hergestellt werden, zur Herstellung eines Polyurethans.

10. Verwendung nach Anspruch 9, dadurch gekennzeichnet, daß das Polyurethan ein Polyurethan-Schaum ist.
